# EUROPEAN PATENT SPECIFICATION

(11) **EP 4 489 697 B1**
(45) Date of publication and mention of the grant of the patent: **18.02.2026**
(21) Application number: 23713433.3
(22) Date of filing: 08.03.2023
(51) Int. Cl.: A61F 2/68, F16D 65/092

(54) **NON-BACKDRIVING ROTATION TRANSMISSION MECHANISM**
NICHT RÜCKDREHENDER ROTATIONSÜBERTRAGUNGSMECHANISMUS
MÉCANISME DE TRANSMISSION DE ROTATION SANS CONTRE-CONDUITE

(30) Priority: 10.03.2022 IT 202200004655
(43) Date of publication of application: 15.01.2025
(73) Proprietor: Fondazione Istituto Italiano di Tecnologia, 16163 Genova (GE) (IT); INAIL - Istituto Nazionale per l'Assicurazione contro gli Infortuni sul Lavoro, 00187 Roma (RM) (IT)
(72) Inventor: MENFI, Giuseppe, 16163 Genova (GE) (IT); GRUPPIONI, Emanuele, 00144 Roma (RM) (IT); STEDMAN, Samuel, 16163 Genova (GE) (IT); BOCCARDO, Nicolò, 16163 Genova (GE) (IT); LAFFRANCHI, Matteo, 16163 Genova (GE) (IT); DE MICHIELI, Lorenzo, 16163 Genova (GE) (IT)
(74) Representative: Bottino, Giovanni
(86) International application number: PCT/IB2023/052199
(87) International publication number: WO 2023/170602

(56) References cited:
- EP-A1- 1 113 184
- EP-A1- 3 865 098
- WO-A2-2004/092606
- WO-A2-2009/066055
- US-A1- 2017 151 071
- CONTROZZI M ET AL: "Miniaturized non-back-drivable mechanism for robotic applications", MECHANISM AND MACHINE THEORY, PERGAMON, AMSTERDAM, NL, vol. 45, no. 10, 1 October 2010 (2010-10-01), pages 1395 - 1406, XP027172435, ISSN: 0094-114X, [retrieved on 20100708]

## Description

The present invention relates to a non-backdriving rotation transmission mechanism comprising an input shaft, an output shaft, a fixed casing having a cylindrical cavity, and provided in said cavity with a cylindrical braking surface, at least one braking element connected to the input shaft and the output shaft and adapted to interact with said braking surface, and means for actuating the braking element. The actuating means are such that the said braking element is movable from a non-braking condition, wherein the transmission of the motion from the input shaft to the output shaft is allowed, to a braking condition, wherein the transmission of the motion from the output shaft to the input shaft is prevented.

In robotics in general, and in particular in the field of anthropomorphic robotics, the development of efficient mechatronic architectures is one of the keys to the success of a functional robotic project. However, this factor becomes even more important in battery-powered systems where the energy saving results in a greater autonomy of work. This is particularly true in the field of myoelectric prostheses. In fact, a prolonged battery life is considered a priority in this type of system and one of the main factors that determine the level of usability of the device.

To achieve increased battery life, one of the most common specifications that a myoelectric prosthesis must have is to be non-backdriving. This means that movement can only be transmitted from the input shaft to the output shaft and not vice versa. This property allows the actuator to resist an external torque without consuming energy from the battery, thus providing the possibility of turning off the motor once the desired position of the joint is reached. Non-backdrivability is very useful in various robotics applications, such as in robotic manipulators, medical exoskeletons or artificial hands or arms and even prosthetic leg joints, and also for safety reasons: power or battery failures should not cause the (potentially dangerous) release of grasped objects or instruments due to backdriving phenomena, nor should they cause the collapse of a patient if a lower limb prosthesis or exoskeleton should lose power supply.

Many non-back-drivable mechanisms have been developed and miniaturized in the past for robotic applications, where weight and size constraints exist. In particular, among the most common examples of these mechanisms are worm gears or screw-nuts. In both mechanisms non-backdrivability is achieved by the shear friction involved during operation, and thus their efficiency is very low: they typically have less than 50% efficiency and often much less. However, these mechanisms are simple to build and assemble, are inexpensive and have been successfully deployed in several prototypes of advanced hand prostheses such as the commercially available Southampton hand, CyberHand and i-Limb.

Further drawbacks of worm gearboxes or screw-nut gearboxes are the intrinsic play at the output, often a high weight, due to the materials used, and consequently a low torque density compared to other actuators. Worm gears can only transmit movement perpendicular to the input, which in robotic applications can be difficult to design, while screw-nut gears can only produce linear movement, which is not always compatible with the intended application. In addition, to prevent overheating, these back-drivable gearboxes often have a low maximum duty cycle, typically 10% at peak torque and 50% at nominal torque, as the friction interface and low efficiency cause the generation of large amounts of heat that can accelerate wear and tear and cause premature failure. Finally, due to the intrinsic friction generated by these devices, one of the two sliding interfaces must be made of a material with low friction, and which is therefore soft and has poor abrasion resistance, such as nylon or bronze. The softer material is designed to wear out slowly with intended use, which leads to increased play over the life of the device until replacement is necessary.

The use of these mechanisms also involves a large overall footprint, which derives from the intrinsically bulky dimensions of the components of the mechanisms themselves.

The document M. Controzzi, C. Cipriani, M. C. Carrozza, "Miniaturized non-back-drivable mechanism for robotic applications", Mechanism and Machine Theory 45 (2010) 1395-1406 describes a miniaturized coupling mechanism for robotic applications, based on the wedge phenomenon in the eccentric-non-eccentric cam coupling. The mechanism comprises a fixed ring, an input shaft with two teeth, an eccentric cam coaxial with the input shaft and fixed to the output shaft, and four brass cylinders which, under the action of two compression springs, tend to wedge between the fixed ring and the cam. When the input shaft is rotated, the teeth unlock the cylinders and an appendage of the two teeth pulls the cam, and then the output shaft. It is not possible to transmit the motion from the output shaft to the drive shaft, as the cylinders block the movement of the cam before the teeth are touched and then dragged.

This mechanism, however, has low torque density and short life, and requires very high manufacturing tolerance requirements which are difficult to achieve in practice.

A further mechanism known to the state of the art is called the Adamant Namiki dyNALOX Locking System, and consists of a passive brake consisting of an input rotor, connected to the output rotor through several steel pins arranged radially. When the input rotor moves, the steel pins are free to rotate along the inside of the outer casing of the mechanism, however when only the output rotates, the specially designed shape of the output cam engages the steel pins between the output cam and the outer casing of the mechanism, thereby creating a braking action that locks the mechanism in the reverse direction.

This mechanism also has low torque density, which necessitates a very high precision required of the components to function, so it is very expensive. The mechanism must also be mounted as an additional gearbox unit, it cannot be integrated for example within a harmonic gearbox since it would damage the flexspline of the latter due to the small contact area of the steel pins.

US 2017/0151071 describes a prosthetic arm having a non-backdriving rotation transmission mechanism with an input cage and an output hexagon cooperating with each other, which engage in a circumferential track by means of locking rollers when a torque is applied from the output towards the input.

This mechanism also has low torque density, necessitates very high precision required of the components to function, so it is very expensive and must be mounted as an additional gearbox unit. The mechanism cannot be integrated inside, for example, a harmonic gearbox, since it would damage the flexspline of the latter due to the small contact area of the steel pins, so it takes up more space.

All devices known in the state of the art have the disadvantage that the steel pins commonly used to wedge against the braking surface have a very small range of movement, which requires a braking surface made with extreme precision.

In addition, steel pins have a very small contact area due to their circular section shape, which induces very high stresses on the braking surface when it is locked; therefore, the braking surface must be made of a very hard material to withstand such contact stresses.

Thus, if the known devices are overloaded, it is relatively easy to permanently deform the braking surface due to the high contact stress. Due to the reduced range of motion of the pins and the high tolerance requirements, even small deformations of the braking surface can damage the mechanism.

The steel pins are always in contact with the outer circular braking surface but rotate forward. However, this contact reduces efficiency.

The object of the present invention is to overcome the aforementioned disadvantages of the currently known mechanisms and to obtain a miniaturizable, low-cost, high-efficiency, back-drivable mechanism that is easy to produce and assemble.

The present invention achieves these objects with a mechanism as disclosed at the beginning, wherein furthermore said braking element comprises two brake pads articulated to one another and each having at least one braking contact zone adapted to interact with said braking surface, which brake pads are movable from an engagement position, corresponding to said braking condition, in which the contact zones are remote from one another and are in contact with said braking surface, to a free position, corresponding to said non-braking condition, in which the contact zones are close to one another and are not in contact with said braking surface.

In an exemplary embodiment, the actuating means comprise at least one spring interposed between the brake pads, which spring is adapted to consistently stimulate the brake pads towards said engagement position.

In an exemplary embodiment, the actuating means comprise an unlocking and locking plate integral with the input shaft and facing the braking element, which unlocking and locking plate is provided with at least one pair of arcuate cam openings, each brake pad being provided with a respective input pin inserted in a corresponding said cam opening.

In one embodiment the radius of curvature of each cam opening is such that the input pin is displaced radially towards the rotational axis of the input shaft in rotational condition of the input shaft while the input pin is displaced radially from said spring towards the braking surface in the absence of rotation of the input shaft. This is preferably enabled by special hollow cams that promote an eccentric radial movement of the brake pads.

In one embodiment, the braking element is connected to the output shaft by means of an engagement hole provided in the articulation fulcrum of the brake pads, the output shaft being provided with an eccentric output pin inserted in said engagement hole.

In a further embodiment, the brake pads are arcuate elements, and each have a first end of mutual constraint and a second free end provided with said braking contact zone.

The eccentric output pin and the geometry of the brake pads cause a self-locking effect with the braking surface since the outer edge of the brake pad, i.e. the braking contact zone, is pushed outwards, i.e. in the direction away from the rotation axis, from the eccentric output pin.

In a preferred embodiment the input shaft and the output shaft are axially aligned with each other.

This is particularly advantageous because the mechanism can be optimally integrated into a pre-existing gearbox where the input and output shafts are concentric and coaxial. Therefore, the mechanism is optimally compatible for integration with epicyclic (planetary) gearboxes, cycloid gearboxes and, above all, harmonic gearboxes (SWG - Strain Wave Gearing).

In a further embodiment the braking surface consists of a tubular element inserted in the cylindrical cavity and constrained to the cylindrical cavity itself.

In one embodiment, the tubular element having the braking surface is at least in part flexible and/or elastically yielding.

The object of the present invention is also a gearbox having coaxial input and output and comprising at least one mechanism as disclosed above, which mechanism is serially connected to said input and/or to said output.

In a preferred embodiment, the gearbox comprises a harmonic gearbox.

Harmonic gearboxes are gearboxes configured to pick up the output motion by exploiting the deformability of a cylindrical element or elastic toothed wheel, typically called a flexspline, which meshes within an outer casing provided with a toothed crown with inner toothing. The flexspline has several teeth fewer than the crown and a smaller diameter than the crown. The meshing between the flexspline and the crown is ensured by a typically elliptical cam, inserted inside the elastic wheel and capable of deforming the latter, so that the toothing of the now deformed flexspline engages with the circular crown with internal toothing. A ball bearing is interposed between the cam and the flexspline to enhance the motion and reduce the friction. The rotating cam performs the function of a carrier assembly and the elastic toothed gear wheel and fixed toothed crown engage internally in two opposite zones. The flexspline therefore ovalizes cyclically, engaging on the fixed crown and rotating in the opposite direction and at a much reduced angular speed with respect to the cam. The flexspline is cup-shaped, with the same axis as the cam connected to the input shaft, so as to transmit the rotational motion to the output.

According to one implementation, the harmonic gearbox includes an intermeshing output flexspline inside an outer casing provided with an internally toothed crown and an input strain wave generating element, the output shaft of the mechanism being connected to said input strain wave generating element, and wherein the mechanism has dimensions such that it is housed at least partly inside said flexspline and the braking element is configured to interact with the braking surface, which braking surface is positioned inside said flexspline.

The mechanism is therefore a passive brake with increased torque density, reduced mechanical play, increased maximum duty cycle and potentially longer duration than classical mechanisms such as worm screw or screw-nut or other mechanisms known from the state of the art.

In addition, being integrated within a harmonic gearbox, the mechanism has in practice zero footprint since the overall volume occupied by the combination of gearbox and mechanism is identical to that occupied by the gearbox alone.

The non-backdrivability is achieved without the need for a secondary actuating device of the braking action, thus also greatly simplifying the control strategies.

The main advantages of the invention are the following:
- The mechanism can be integrated into gearboxes to create an ultra-compact actuator with very high braking torque without changing the external dimensions;
- Very high torque density compared to known state-of-the-art mechanisms due to the self-locking effect disclosed above;
- Greater efficiency in the forward motion thanks to the condition of zero contact between the brake pad and the braking surface, while the steel pins used in the known mechanisms are always in contact with the external braking surface;
- Greater range of motion of the brake pads, which move by several millimetres during locking and unlocking, compared to the steel pins used in the known mechanisms, which move by a fraction of a millimetre. This means that the design of the mechanism according to the present invention is less sensitive to manufacturing tolerances and the external braking surface does not have to be perfectly circular, so the mechanism is much cheaper to produce and easier to assemble. Furthermore, this allows use of a soft and/or flexible braking surface.
- The greater range of motion of the brake pads also means that the design is much more tolerant to damage suffered by the braking surface during use, as there is more play when the brake pads are in the free position, resulting in greater durability of the entire mechanism.
- The greater range of motion of the brake pads also allows the braking surface to be fixed inside the flexspline of a harmonic gearbox, which flexspline oscillates for several millimetres in the radial direction during use. The steel pins used in the prior art require a precise circular surface to function. In addition, the small contact zone of the steel pins would damage the flexspline, which is typically made of very thin steel sheet, so it cannot withstand high contact stresses.
- The design of the mechanism is relatively simple, it has a lower number of parts compared to the mechanisms known in the state of the art and it is easy to assemble compared to the mechanisms known in the state of the art, due to the lack of more steel pins and their springs.
- Reduced play at the output.
- Negligible noise during operation.
- The brake pads have a much wider contact area with the braking surface than the steel pins used in the mechanisms known from the state of the art. The wider contact area means that damage to the braking surface is much less likely to occur during overloading of the brake output, due to reduced localized contact stress.
- When the mechanism is integrated in a gearbox, the braking torque is multiplied by the gear ratio of the gearbox, in the case of a harmonic gearbox between 30 and 150 times higher.
- When the mechanism is integrated into a gearbox, the forces on the braking surface are generally reduced, leading to an even longer life.
- When the mechanism is integrated in a harmonic gearbox, the play is eliminated at the output.

Finally, when the mechanism is integrated within a harmonic gearbox, it is extremely advantageous to combine high forward and non-backdriving efficiency (i.e. zero reverse efficiency) with zero output play, especially for the fields of industrial robotic manipulation, mobile robots with legs, robotic lower limb prostheses and robotic upper limb prostheses.

These and other features and advantages of the present invention will become clearer from the following disclosure of some non-limiting embodiments shown in the attached drawings, wherein:
Fig. 1 shows an exploded view of a preferred embodiment;
Fig. 2 shows a sectional view of the brake pads;
Fig. 3 shows a sectional view of the unlocking and locking plate, wherein the cammed grooves are present;
Figs. 4a and 4b respectively show the braking condition and the non-braking condition;
Figs. 5a, 5b and 5c show the transition from the braking condition to the non-braking condition;
Fig. 6 shows the dimensional relationships between the unlocking and locking plate and the brake pads;
Figs. 7a and 7b show respectively the braking condition and a detailed view of the braking contact zone;
Figs. 8a and 8b show the self-locking effect in an automotive drum brake;
Fig. 9 shows the dynamic components of the self-locking effect;
Figs. 10a and 10b show two embodiments with a double pair of brake pads;
Fig. 11a shows an embodiment example of the mechanism previously disclosed with braking force acting on the external body;
Fig. 11b shows the mechanism integrated in an epicyclic or cycloidal gearbox with braking force acting on the outer ring or body;
Fig. 11c shows the mechanism integrated in an epicyclic or cycloidal gearbox with braking force acting between input and output carrier;
Fig. 11d shows the mechanism integrated in a strain gearing gearbox with braking force acting between input and output;
Fig. 12 and Figs. 13a and 13b show sectional views of an embodiment example of the mechanism integrated in a harmonic gearbox;
Fig. 14 shows an exploded view of a further exemplary embodiment of the mechanism.

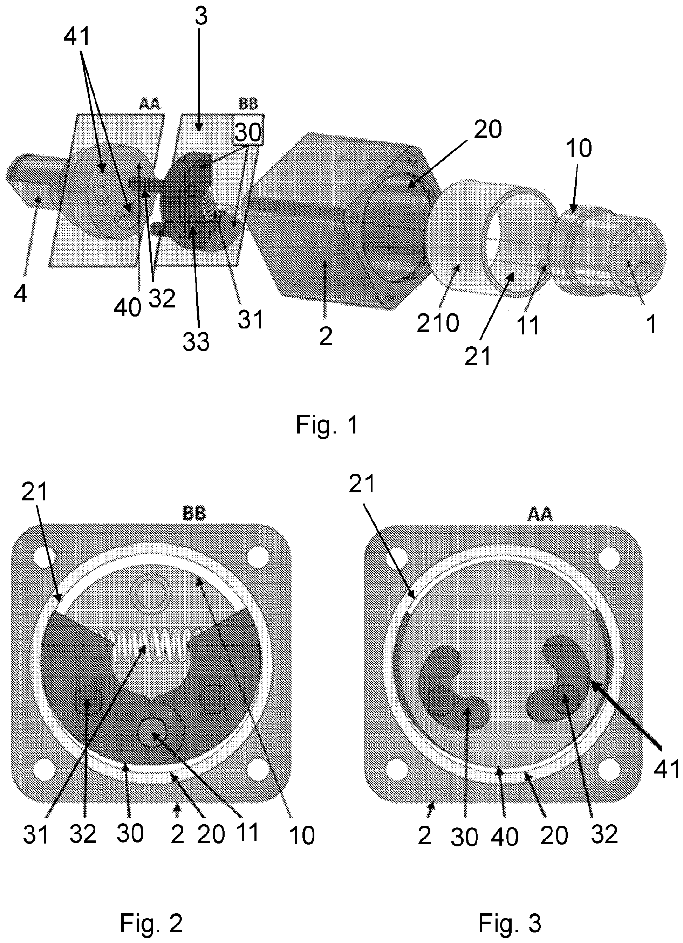

The exploded Figure 1 shows a preferred embodiment of the non-backdriving rotation transmission mechanism according to the present invention. The mechanism comprises an input shaft 4, an output shaft 1, a fixed outer casing 2 having a cylindrical cavity 20 and provided in said cavity with a cylindrical braking surface 21 and at least one braking element 3 connected to the input shaft 4 and to the output shaft 1 and adapted to interact with the braking surface 21.

The input shaft 4, the output shaft 1 and the cylindrical cavity 20, and consequently the cylindrical braking surface 21, are coaxial with each other.

The braking surface 21 preferably consists of a tubular element 210 inserted in the cylindrical cavity 20 and constrained to the cylindrical cavity 20 itself.

Actuating means of the braking element 4 are provided such that the braking element 4 is movable from a braking operating condition (back-drive), wherein transmission of the motion from the output shaft 1 to the input shaft 4 is prevented, to a non-braking operating condition (forward-drive), wherein transmission of the motion from the input shaft 4 to the output shaft 1 is allowed.

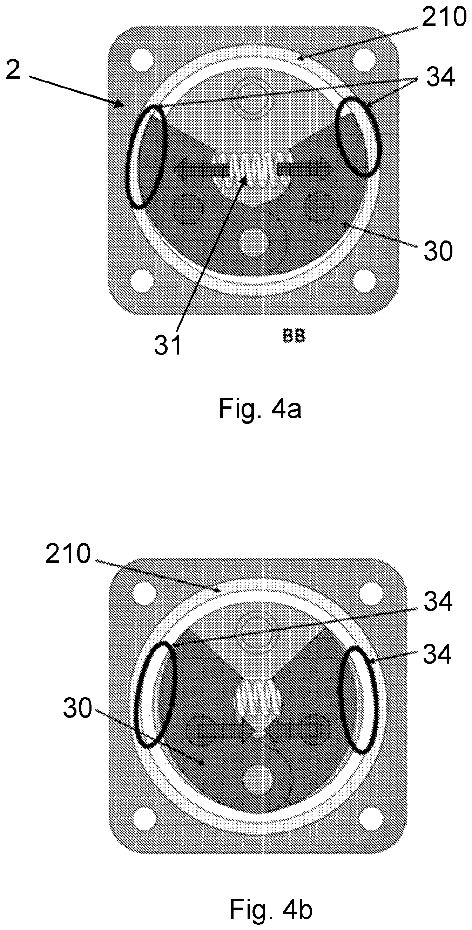

The braking element 3 comprises two brake pads 30 articulated to each other and each having a respective braking contact zone 34, which braking contact zones are shown in detail in Figures 4a and 4b and are adapted to interact with the braking surface 21.

The brake pads 30 are hinged arcuate elements, and each has a first end of mutual constraint and a second free end provided with the braking contact zone 34.

The braking element 3 is connected to the output shaft 1 by means of an engagement hole 33 provided in the articulation fulcrum of the brake pads 30, the output shaft 1 being provided with an eccentric output pin 11 inserted in the engagement hole 33. The output pin 11 is preferably supported by an output disc 10 located at the proximal end of the output shaft 1 and integral thereto.

As illustrated in Figures 2 and 3, the actuating means comprise at least one spring 31 interposed between the brake pads 30, which spring 31 is adapted to consistently stimulate the brake pads 30 towards said engagement position.

The actuating means further comprise an unlocking and locking plate 40 integral to the input shaft 4 and facing the braking element 3. The unlocking and locking plate 40 is provided with at least one pair of arcuate cam openings 41, in particular consisting of arcuate slots or openings made in the unlocking and locking plate 40. Each brake pad 30 is provided with a respective input pin 32 inserted in a corresponding said cam opening 41.

The unlocking and locking plate is preferably disc-shaped, but can have any shape such as to allow the cam openings 41 to be positioned eccentrically.

Figures 2, 3, 4a, 4b, 5a, 5b and 5c show sectional views perpendicular to the axis of rotation in section planes AA and BB shown in Figure 1.

The core of the invention is therefore based on the coupling of two or more brake pads 30, a fixed outer ring consisting of the casing 2 and an output shaft 1.

As shown in Figures 4a and 4b, the brake pads 30 are movable between two extreme positions. In Figure 4a the brake pads 30 are in an engagement (or open) position, corresponding to said braking condition, in which the contact zones 34 are spaced apart from each other and are in contact with the braking surface 21. In Figure 4b the brake pads 30 are in a free (or closed) position, corresponding to the non-braking operating condition, in which the contact zones 34 are close to each other and are not in contact with the braking surface 21.

Therefore, the connection between the braking element 3 and the output shaft 1, guaranteed by the engagement hole 33 at the hinge constraint point between the brake pads 30 and the eccentric output pin 2, allows each brake pad 30 to act as a brake in the fully engaged or open position, where each brake pad 30 comes into contact with the external braking surface 21, a condition known in the jargon as "sticking", or as a transmission component in the free or fully closed position, where each brake pad 30 is not in contact with the external braking surface 21.

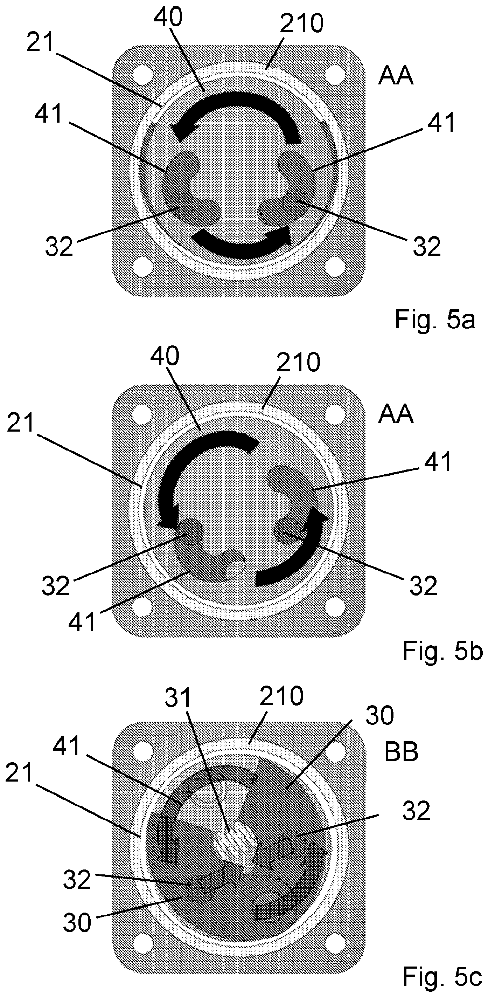

Figures 5a, 5b and 5c show with different views the unlocking action of the actuating means against the braking element 3.

The spring 31 ensures that the braking element is always in the engagement position, i.e. maximum opening, in the absence of input torque.

The radius of curvature of each cam opening 41 is such that the input pin 32 is displaced radially towards the rotational axis of the input shaft 4 in the rotational condition of the input shaft 4, while the input pin 32 is displaced radially from the spring 31 towards the braking surface 21 in the absence of rotation of the input shaft 4.

The default position of the mechanism is therefore that of engagement, shown in Figure 5a, thanks to the presence of the pre-loaded spring 31, which consistently stimulates the brake pads 30 in the direction of separation from one another, as indicated by the arrows in Figure 4a. A torque applied to the input shaft 4 causes the two brake pads 30 to close inwardly, as indicated by the arrows shown in Figure 4b. This is ensured by the fact that the input pins 32 are constrained to move inwards, i.e. towards the axis of rotation of the input shaft 4, by the arcuate cam openings 41 provided in the unlocking and locking plate 40 integral with the input shaft 4, which cam openings guide the input pins 32 fixed to the brake pads 30.

When the brake pads 30 close inwardly, i.e. move to the free position, their braking contact zones 34 separate and move away from the fixed ring braking surface 21. Therefore, the input torque is transferred from the input shaft 4 to the eccentric output pin 11 and then to the output shaft 1. Thus, in this case, the mechanism does not provide any braking force due to the loss of contact with the braking surface 21 of the casing 2 and transmits the rotation motion with a very high efficiency.

In summary:
a. The input shaft 4 is rotated by the torque provided by an external motor;
b. The rotation of the input shaft 4 forces the input pins 32 to move inward due to the unlocking slots or openings 41 provided in the unlocking and locking plate 40 integral to the input shaft 4;
c. The input pins 32, moving inwardly, cause the brake pads 30 on which they are fixed to rotate inwardly about the hinge point, in which the output pin 11 is coupled;
d. The brake pads 30, held until then against the braking surface 21 by the pre-loaded spring 31, lose contact with the fixed-ring braking surface 21 of the casing 2;
e. Once the brake pads 30 lose contact, the output pin 11 connected to the output shaft 1 is free to move and then the output shaft starts to rotate due to the input torque.

In a further embodiment, the spring 31 or the springs 31 can be dimensioned in such a way as to identify a transient phase between the braking condition and the non-braking condition, in which transient phase the brake pads 30 are in the engagement position but slide slightly on the braking surface 21, transferring only a part of the torque from the input shaft to the output shaft. The mechanism thus acts as a clutch and, preferably for some input torque ranges, transmits a fraction of the input torque towards the output.

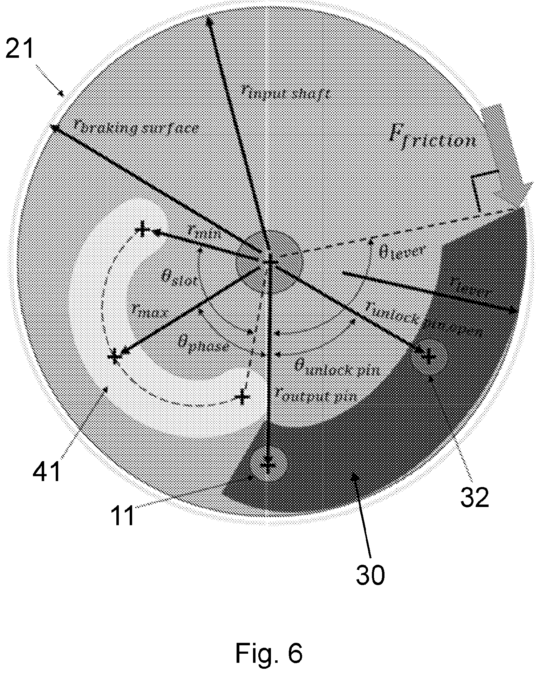

Figure 6 shows the geometric dimensional requirements of the cam openings 41 for unlocking the brake pads 30.

The dimensional ranges are preferably as follows.
*θₚₕₐₛₑ*: any angle, optimally 60°-120°
*θₛₗₒₜ*: 10°-90°
*r_{input shaft}*: any radius
*r_{output pin}*: ~ 0.8 × *r_{output disk}*
*rₘᵢₙ*: 0.3 *r_{output pin}* to 1.5 *r_{output pin}*
*rₘₐₓ*: 1.01 *rₘᵢₙ* to 1.5 *rₘᵢₙ*
*u_{nlock pin,open}*: *rₘₐₓ*
*θ_{unlock pin}*: *θₚₕₐₛₑ*
*r_{braking surface}*: any radius
*rₗₑᵥₑᵣ*: ≤ *r_{braking surface}*
*θₗₑᵥₑᵣ*: ≥ 90° for the self-locking effect to occur

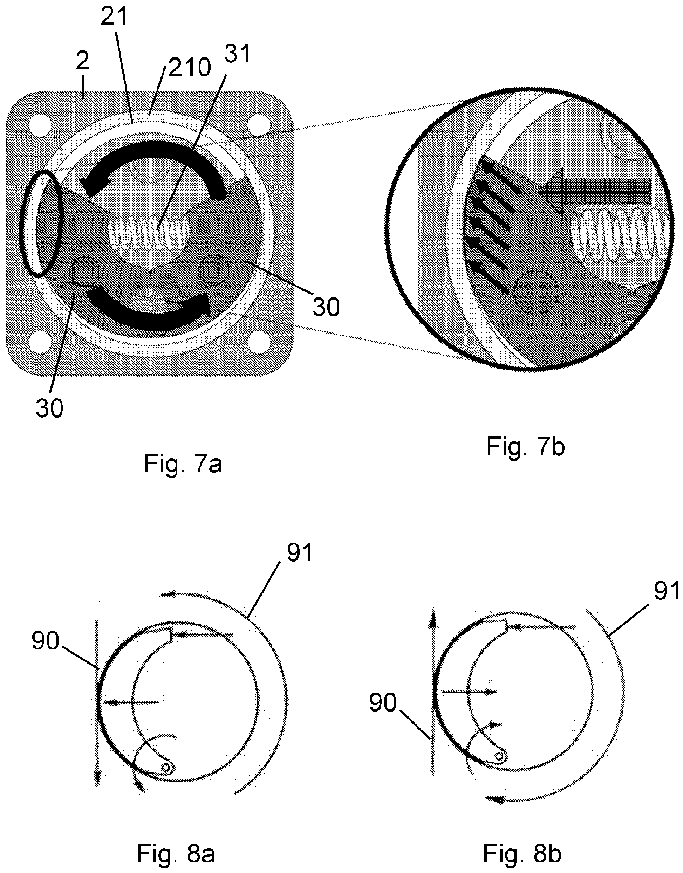

Figure 7a shows the braking element 3 in braking condition, i.e. in the case of backdriving.

When an external backdriving torque is applied to the output shaft 1, the torque is transmitted from the output shaft 1 to the eccentric output pin 11 connected to each brake pad 30, which is in the engaged position, i.e. fully open, due to the presence of the pre-loaded compression spring 31, which also strongly reduces the play. At this point, the eccentric output pin 11 and the geometry of the brake pad 30 cause a self-locking effect with the braking surface 21 since the outer edge of the brake pad 30, i.e. the braking contact zone 34, is pushed outwardly, i.e. in a direction away from the rotation axis, from the eccentric output pin 11. This configuration of use is defined in the jargon as a "jamming" brake.

Preferably, the braking surface 21 is made of or covered with an abrasion-resistant but high-friction material to increase the braking effect.

The outward thrust of the brake pads 30 locks the output shaft 1 to the fixed outer casing 2, thus disabling the transfer of the backdriving torque.

It is important to note that in the configuration shown in Figure 7a and in detail in Figure 7b the self-locking effect is experienced only by the brake pad 30 on the left in the figure during a clockwise rotation and only by the brake pad 30 on the right in the figure during an anticlockwise rotation.

As seen in Figures 8a and 8b, this is due to the fact that the force applied to the brake pad 30 by the eccentric output pin 11 must be in the opposite direction to the braking force due to friction, for the self-locking effect (or self-servo) to occur.

The self-locking effect also occurs only when the combination of the coefficient of friction and the angle of the frictional force is sufficiently high. Assuming a coefficient of friction of 1, the self-locking effect reliably occurs when the value of the parameter *θ_lever,* shown in Figure 6, is greater than 90°. The mechanism will still apply a braking torque to the output shaft 1 for values below 90°, however the reliability of the brake will be greatly reduced.

Figs. 8a and 8b show how the direction of the frictional force 90 in an automotive type drum brake varies when the direction of rotation 91 of the drum is reversed.

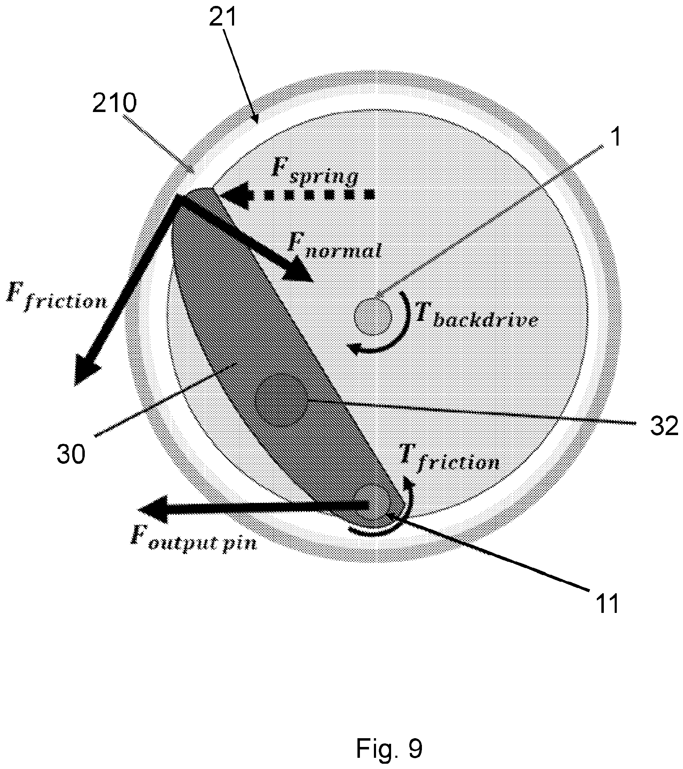

Figure 9 shows the backdriving force diagram, which shows that the backdriving torque causes a self-locking force reaction (self-servo) involving only one of the two brake pads 30. When the output shaft 1 rotates in a clockwise direction due to a torque *T_backdrive,* the force of the output pin 11 on the brake pad 30 rotates said brake pad 30 in a clockwise direction, which due to the preloading of the spring 31 *F_spring* is already in contact with the external braking surface 21. The arrow of the force of the spring 31 is dashed because after the initial contact with the outer braking surface 21, the force of the spring 31 makes a negligible contribution to the generated braking force. The friction force *F_friction* generated by this initial contact induces a counter-clockwise moment *T_friction* on the brake pad 30 around the output pin 11. Such counter-clockwise moment *T_friction* increases the normal reaction force applied by the external braking surface 21 to the brake pad 30, further increasing the braking force until the whole mechanism stops rotating and reaches static equilibrium.

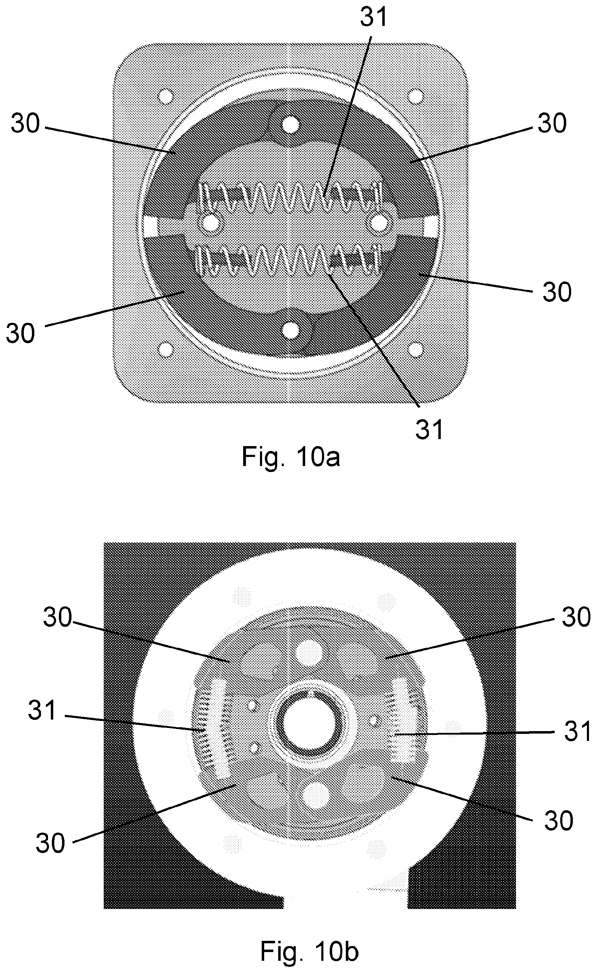

Figures 10a and 10b show two embodiments of the mechanism, wherein the braking element 3 comprises two pairs of brake pads 30.

Although all of the above Figures show only one pair of brake pads 30, multiple pairs of brake pads 30 may be used. This offers several advantages. First, the braking torque is increased due to the larger surface area of the braking interface. In addition, depending on the available range of motion (ROM) and torque requirements, two or more pairs of brake pads 30 may be provided to provide a perfect balance of the radial mass of the mechanism, thereby reducing vibrations during high speed operation. In Figures 10a and 10b two different versions of a mechanism provided with a double pair of brake pads 30 are shown. The operation principle of each passive brake with multiple pairs of brake pads 30 is identical to that disclosed above and the components involved are the same, however the length of each brake pad 30 can be reduced and the number of contact points between the braking surface 21 and the brake pads 30 is increased.

Another important feature of the back-drivable mechanism according to the present invention is that it can be integrated within a pre-existing gearbox, such as a gearmotor.

Depending on the configuration of the gearbox, the mechanism may be integrated in such a way that the braking force acts on the outer casing 2 or on the ring of the gearbox, or it may be designed to block both the input and output of the gearbox. Since all gearboxes by their intrinsic nature increase or decrease the rotation speed between the input and the output, the integral blocking of the input and the output will result in the blocking of the mechanism if the output shaft 1 rotates without a corresponding proportional rotation of the input shaft 4.

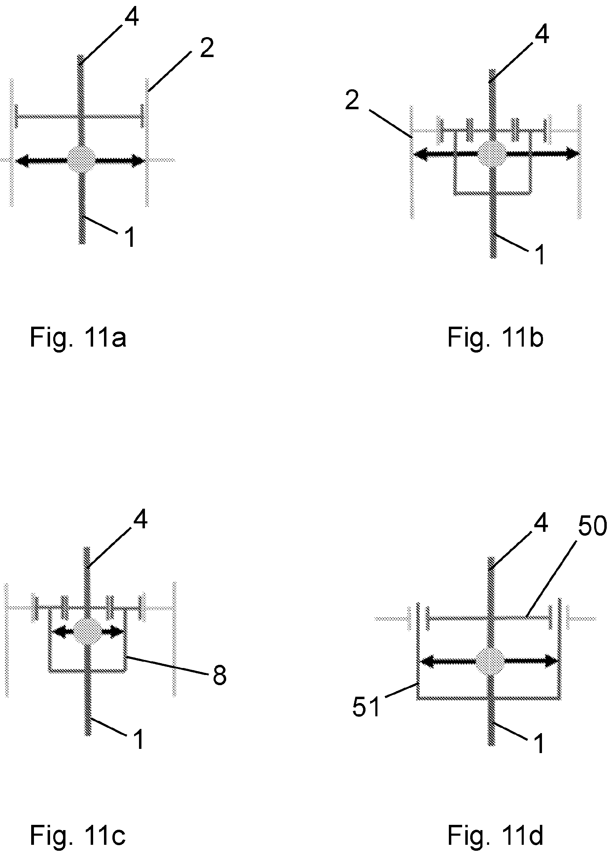

Figures 11a, 11b, 11c and 11d show different embodiments of use of the mechanism that is the object of the present invention, including with the integration of the mechanism itself in different gear boxes.

Figure 11a shows a schematic of the mechanism as detailed above, without any integration into a gearbox and with a braking force acting between input shaft 4, output shaft 1 and outer casing 2.

Figure 11b shows a schematic of the mechanism integrated in an epicyclic or cycloidal gearbox with a braking force acting on the outer casing 2.

Figure 11c shows a schematic of the mechanism integrated in an epicyclic or cycloidal gearbox with a braking force acting between input shaft 4 and output of the carrier 8.

Figure 11d shows a schematic of the mechanism integrated in a harmonic gearbox (SWG - Strain Wave Gearing) with a braking force acting between input wave generator 50 and output 51.

Thus, the mechanism is compatible for integration with epicyclic (planetary) gearboxes using cylindrical or helical teeth or frictional planetary elements in the configurations of Figs. 11b and 11c. **In** these configurations, the mechanism is also compatible for incorporation into a cycloid gearbox. Finally, and above all, the mechanism is compatible for integration with strain wave gearboxes, or harmonic gearboxes, in the form of Figure 11d. In this configuration, the entire mechanism can fit within the existing volume of the harmonic gearbox, resulting in a highly compact and efficient gearbox, which has an excellent torque density but is non back-drivable.

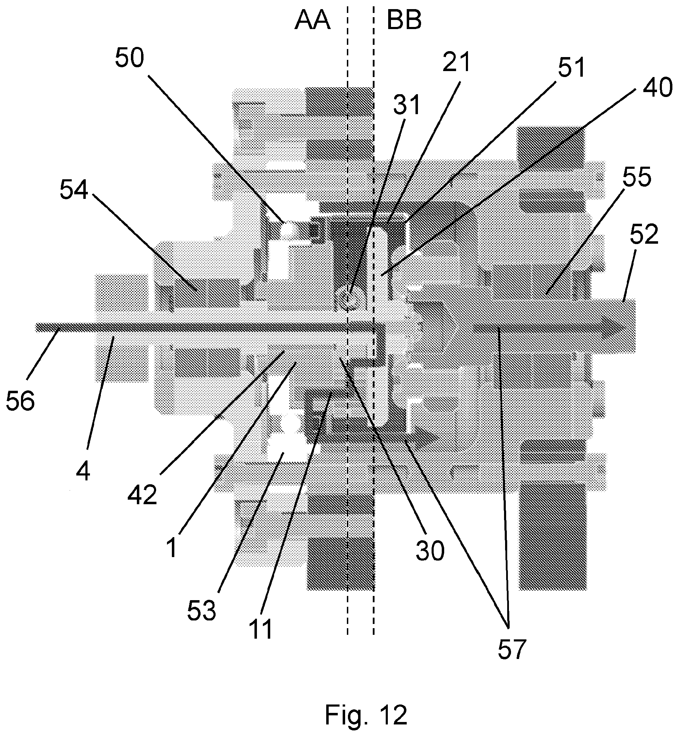

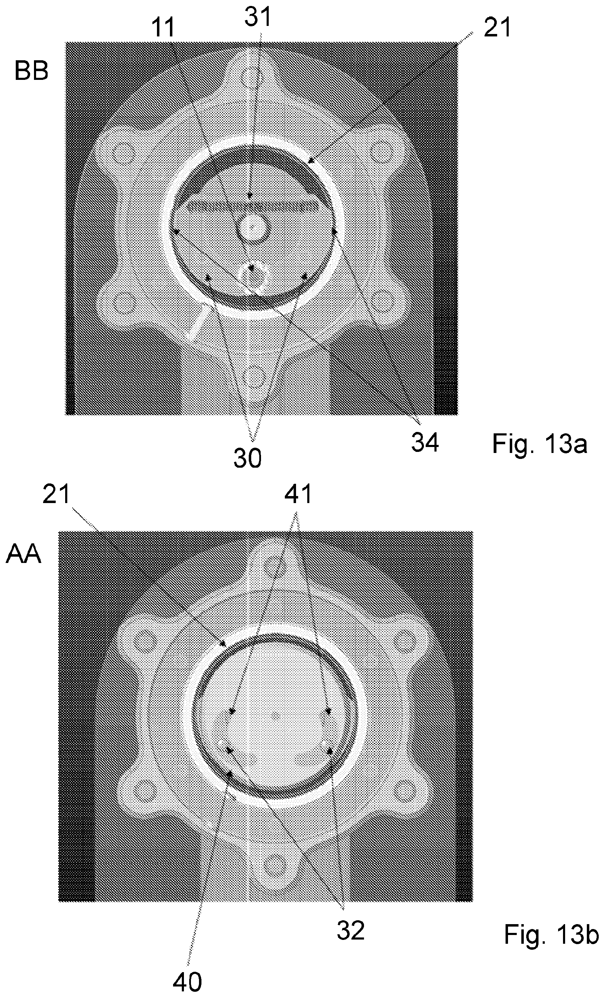

In order for the configuration of Figure 11d to function, the mechanical arrangement of the mechanism disclosed above must be significantly modified to incorporate it within a harmonic gearbox, as shown in Figures 12, 13a and 13b.

Figure 12 shows a longitudinal sectional view, i.e. along the axis of rotation, of the mechanism integrated in a commercial type of harmonic gearbox. In this exemplary embodiment, the mechanism utilizes a flexible braking surface 21. Figures 13a and 13b show sectional views perpendicular to the axis of rotation in the section planes AA and BB shown in Figure 12.

The harmonic gearbox includes an intermeshing output flexspline 51 within an outer casing 53 provided with an internally toothed crown and an input strain wave generating element 50. The output shaft 1 of the mechanism is connected to the strain wave generating element 50. The mechanism is dimensioned such that it is housed partly within the flexspline 51. The braking element 3 is configured to interact with the braking surface 21, which braking surface 21 is placed inside the said flexspline 51.

Due to the internal arrangement of the harmonic gearbox, therefore, the mechanism lies in the void volume that exists in every harmonic gearbox downstream of the strain wave generator 50. Therefore, the output shaft 1 of the mechanism, directly integrated into the strain wave generator 50 of the harmonic gearbox, faces the input side. This is the opposite of the mechanism disclosed above and is obtained by providing an output shaft 1 coaxial to the input shaft 4 and supported by the input shaft 4 itself by means of a bearing 42.

As can be seen in Figure 12, the torque from the input shaft 1 is first transferred to the input disc constituting the unlocking and locking plate 40, then, by going back, this input torque is transferred to the two or more brake pads 30 via the two or more input pins 32, which are forced to move in the unlocked position by the cam openings 41 of the unlocking and locking plate 40. The input torque is then transferred from the brake pads 30 to the output pin 11, which is itself directly connected to the strain wave generator 50 of the harmonic gearbox. Once the input torque has been transferred to the strain wave generator 50, the input torque in the harmonic gearbox is multiplied by the reduction ratio as usual and transferred to the output shaft 52 of the harmonic gearbox via the flexspline 51.

The fast axis is indicated in the Figure with reference numeral 56 and the slow axis with reference numeral 57.

When the harmonic gearbox is backdriven, the backdriving torque is transferred from the flexspline 51 to the strain wave generator 50 and subsequently to the output pin 11. The output pin 11 forces the brake pads 30 to move outwards in the engagement position and thus to come into contact with the braking surface 21 provided inside the flexspline 51, in a manner similar to the autonomous passive brake explained above. This has the effect of interrupting the input and output of the harmonic gearbox, effectively blocking the harmonic gearbox under the effect of the backdriving torque.

The braking surface 21 is advantageously constituted by a separate flexible element 210 and fixed inside the flexspline 51.

The input 4 and output shafts 52 of the entire gearbox are supported by input 54 and output 55 bearings.

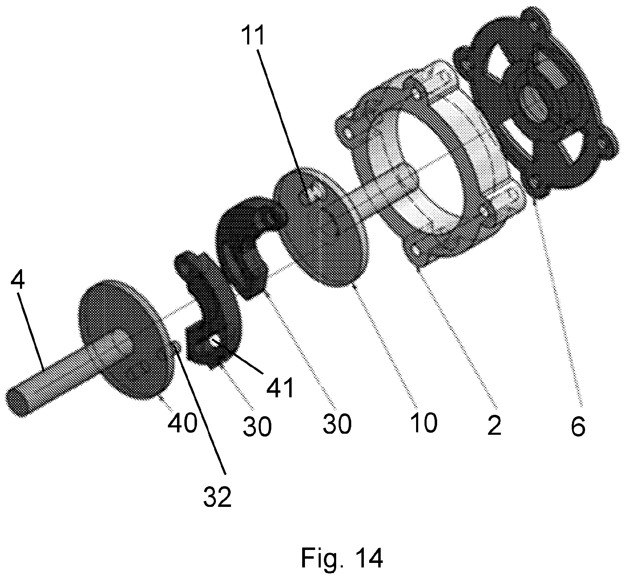

Figure 14 shows an embodiment variant in which the input pins 32, instead of being fixed to the brake pads 30, are fixed to the input shaft 4, in particular to the end disc or plate of the input shaft 4. The cam openings 41 are instead provided on the brake pads 30.

Similarly, instead of providing an eccentric output pin 11 fixed to the output shaft 1, it is possible to provide an engagement hole eccentrically provided in a terminal of the output shaft and correspondingly provide the eccentric output pin fixed to the braking element 3.

The mechanism that is the object of the present invention finds application in the following industrial sectors:
- Industrial automation as a safety device
- Industrial robotic arms as an energy saving device
- Prosthetic devices as an energy and weight saving device
- Medical exoskeletons
- Industrial exoskeletons
- Humanoid and legged robots
- Safety brake for lifting devices such as winches, winches or cranes.
- Any other application in which a high-efficiency forward rotation transmission is required, while backdriving is to be prevented.

## Claims

1. Non-backdriving rotation transmission mechanism comprising an input shaft (4), an output shaft (1), a fixed casing (2) having a cylindrical cavity (20), and provided in said cavity (20) with a cylindrical braking surface (21), at least one braking element (3) connected to the input shaft (4) and to the output shaft (1) and adapted to interact with said braking surface (21), and means for actuating the braking element (3) such that said braking element (3) is movable from a non-braking condition, wherein transmission of the motion from the input shaft (4) to the output shaft (1) is allowed, to a braking condition, wherein transmission of the motion from the output shaft (1) to the input shaft (4) is prevented,
**characterized in that**
the said braking element (3) comprises two brake pads (30) articulated to each other and each having at least one braking contact zone (34) adapted to interact with said braking surface (21), which brake pads (30) are movable from an engagement position, corresponding to said braking condition, wherein the contact zones (34) are remote from each other and are in contact with said braking surface (21), to a free position, corresponding to said non-braking condition, wherein the contact zones (34) are close to each other and are not in contact with said braking surface (21).

2. Transmission mechanism according to claim 1, wherein the actuating means comprise at least one spring (31) interposed between two said brake pads (30), which spring (31) is adapted to consistently stimulate the brake pads (30) towards said engagement position.

3. Transmission mechanism according to claim 2, wherein the actuating means comprise an unlocking and locking plate (40) integral with the input shaft (4) and facing the braking element (3), which unlocking and locking plate (40) is provided with at least one pair of arcuate cam openings (41), each brake pad (30) being provided with a respective input pin (32) inserted in one corresponding said cam opening (41).

4. Transmission mechanism according to claim 3, wherein the radius of curvature of each cam opening (41) is such that the respective input pin (32) is displaced radially towards the rotational axis of the input shaft (4) in the rotational condition of the input shaft (4), while the input pin (32) is displaced radially from the said spring (31) towards the braking surface (21) in the absence of rotation of the input shaft (4).

5. Transmission mechanism according to one or more of the preceding claims, wherein the braking element (3) is connected to the output shaft (1) by means of an engagement hole (33) provided in the articulation fulcrum of the brake pads (30), the output shaft (1) being provided with an eccentric output pin (11) inserted in said engagement hole (33).

6. Transmission mechanism according to one or more of the preceding claims, wherein the brake pads (30) are arcuate elements, and each have a first reciprocally constraining end and a second free end provided with said braking contact zone (34).

7. Transmission mechanism according to one or more of the preceding claims, wherein the input shaft (4) and the output shaft (1) are axially aligned with each other.

8. Transmission mechanism according to one or more of the preceding claims, wherein the braking surface (21) consists of a tubular element inserted in the cylindrical cavity (20) and constrained to the cylindrical cavity (20) itself.

9. Gearbox having coaxial input and output,
**characterized in that**
it comprises at least one mechanism according to one or more of the preceding claims, which mechanism is connected in series to said input and/or to said output.

10. Gearbox according to claim 9 comprising a harmonic gearbox, which harmonic gearbox includes an intermeshing output flexspline (51) within an outer casing (53) provided with an internally toothed crown and an input strain wave generating element (50), the output shaft (1) of the mechanism being connected to said input strain wave generating element (50), and wherein the mechanism has dimensions such that it is housed at least partly within said flexspline (51) and the braking element (3) is configured to interact with the braking surface (21), which braking surface (21) is placed within said flexspline (51).

## Patentansprüche

1. Nicht rückwirkender Rotationsübertragungsmechanismus, umfassend eine Eingangswelle (4), eine Ausgangswelle (1), ein feststehendes Gehäuse (2) mit einem zylindrischen Hohlraum (20), der in dem Hohlraum (20) mit einer zylindrischen Bremsfläche (21) versehen ist, mindestens ein Bremselement (3), das mit der Eingangswelle (4) und der Ausgangswelle (1) verbunden ist und mit der Bremsfläche (21) zusammenwirkt, und Mittel zum Betätigen des Bremselements (3), so dass das Bremselement (3) aus einem NichtBremszustand, in dem die Übertragung der Bewegung von der Eingangswelle (4) auf die Abtriebswelle (1) möglich ist, in einen Bremszustand, in dem die Übertragung der Bewegung von der Abtriebswelle (1) auf die Antriebswelle (4) verhindert wird,
**dadurch gekennzeichnet, dass**
das Bremselement (3) zwei miteinander gelenkig verbundene Bremsbeläge (30) umfasst, die jeweils mindestens eine Bremskontaktzone (34) aufweist, die mit der Bremsfläche (21) zusammenwirken kann, wobei die Bremsbeläge (30) aus einer Eingriffsposition, die dem Bremszustand entspricht, in der die Kontaktzonen (34) voneinander entfernt sind und mit der Bremsfläche (21) in Kontakt stehen, in eine Freigabeposition, die dem Nichtbremszustand entspricht, bewegbar sind, in der die Kontaktzonen (34) nahe beieinander liegen und nicht mit der Bremsfläche (21) in Kontakt stehen.

2. Übertragungsmechanismus nach Anspruch 1, wobei die Betätigungsmittel mindestens eine Feder (31) umfassen, die zwischen zwei der Bremsbeläge (30) angeordnet ist, wobei die Feder (31) dazu ausgelegt ist, die Bremsbeläge (30) kontinuierlich in Richtung der Eingriffsposition zu stimulieren.

3. Übertragungsmechanismus nach Anspruch 2, wobei die Betätigungsmittel eine Entriegelungs- und Verriegelungsplatte (40) umfassen, die mit der Eingangswelle (4) einstückig ist und dem Bremselement (3) zugewandt ist, wobei die Entriegelungs- und Verriegelungsplatte (40) mit mindestens einem Paar bogenförmiger Nockenöffnungen (41) versehen ist, wobei jedes Bremsbelag (30) mit einem entsprechenden Eingangsstift (32) versehen ist, der in eine entsprechende Nockenöffnung (41) eingeführt ist.

4. Übertragungsmechanismus nach Anspruch 3, wobei der Krümmungsradius jeder Nockenöffnung (41) so bemessen ist, dass der jeweilige Eingangsstift (32) im Drehzustand der Eingangswelle (4) radial in Richtung der Drehachse der Eingangswelle (4) verschoben wird, während der Eingangsstift (32) bei fehlender Drehung der Eingangswelle (4) radial von der Feder (31) in Richtung der Bremsfläche (21) verschoben wird.

5. Übertragungsmechanismus gemäß einem oder mehreren der vorstehenden Ansprüche, wobei das Bremselement (3) mit der Abtriebswelle (1) mittels einer Eingriffsbohrung (33) verbunden ist, die im Gelenkpunkt der Bremsbeläge (30) vorgesehen ist, wobei die Abtriebswelle (1) mit einem exzentrischen Abtriebsstift (11) versehen ist, der in die Eingriffsbohrung (33) eingeführt ist.

6. Übertragungsmechanismus gemäß einem oder mehreren der vorstehenden Ansprüche, wobei die Bremsbeläge (30) bogenförmige Elemente sind und jeweils ein erstes, gegenseitig begrenzendes Ende und ein zweites freies Ende aufweisen, das mit der Brems-Kontaktzone (34) versehen ist.

7. Übertragungsmechanismus gemäß einem oder mehreren der vorstehenden Ansprüche, wobei die Eingangswelle (4) und die Ausgangswelle (1) axial zueinander ausgerichtet sind.

8. Übertragungsmechanismus gemäß einem oder mehreren der vorstehenden Ansprüche, wobei die Bremsfläche (21) aus einem rohrförmigen Element besteht, das in den zylindrischen Hohlraum (20) eingesetzt und an den zylindrischen Hohlraum (20) selbst gebunden ist.

9. Getriebe mit koaxialem Eingang und Ausgang,
**dadurch gekennzeichnet, dass**
es mindestens einen Mechanismus gemäß einem oder mehreren der vorstehenden Ansprüche umfasst, wobei dieser Mechanismus in Reihe mit dem Eingang und/oder dem Ausgang verbunden ist.

10. Getriebe gemäß Anspruch 9, das ein harmonisches Getriebe umfasst, wobei das harmonische Getriebe eine ineinandergreifende Flexspline-Ausgangswelle (51) innerhalb eines Außengehäuses (53) mit einer innenverzahnten Krone und einem Eingangs-DehnungswellenErzeugungselement (50) umfasst, wobei die Ausgangswelle (1) des Mechanismus mit dem Eingangs-Dehnungswellenerzeugungselement (50) verbunden ist und wobei der Mechanismus solche Abmessungen aufweist, dass er zumindest teilweise innerhalb der Flexspline (51) untergebracht ist und das Bremselement (3) so konfiguriert ist, dass es mit der Bremsfläche (21) zusammenwirkt, wobei die Bremsfläche (21) innerhalb der Flexspline (51) angeordnet ist.

## Revendications

1. Mécanisme de transmission à rotation sans retour comprenant un arbre d'entrée (4), un arbre de sortie (1), un carter fixe (2) comportant une cavité cylindrique (20) et muni dans ladite cavité (20) d'une surface de freinage cylindrique (21), au moins un élément de freinage (3) relié à l'arbre d'entrée (4) et à l'arbre de sortie (1) et adapté pour interagir avec ladite surface de freinage (21), et des moyens pour actionner l'élément de freinage (3) de telle sorte que ledit élément de freinage (3) soit mobile à partir d'une condition de non-freinage, dans laquelle la transmission du mouvement de l'arbre d'entrée (4) vers l'arbre de sortie (1) est autorisée, à une condition de freinage, dans laquelle la transmission du mouvement de l'arbre de sortie (1) vers l'arbre d'entrée (4) est empêchée,
**caractérisé en ce que**
ledit élément de freinage (3) comprend deux plaquettes de frein (30) articulées l'une à l'autre et comportant chacune au moins une zone de contact de freinage (34) adaptée pour interagir avec ladite surface de freinage (21), lesdites plaquettes de frein (30) étant mobiles depuis une position d'engagement, correspondant à ladite condition de freinage, dans laquelle les zones de contact (34) sont éloignées l'une de l'autre et sont en contact avec ladite surface de freinage (21), vers une position libre, correspondant à ladite condition de non-freinage, dans laquelle les zones de contact (34) sont proches l'une de l'autre et ne sont pas en contact avec ladite surface de freinage (21).

2. Mécanisme de transmission selon la revendication 1, dans lequel les moyens d'actionnement comprennent au moins un ressort (31) interposé entre les deuxdites plaquettes de frein (30), lequel ressort (31) est adapté pour stimuler de manière constante les plaquettes de frein (30) vers ladite position d'engagement.

3. Mécanisme de transmission selon la revendication 2, dans lequel les moyens d'actionnement comprennent une plaque de déverrouillage et de verrouillage (40) solidaire de l'arbre d'entrée (4) et faisant face à l'élément de freinage (3), laquelle plaque de déverrouillage et de verrouillage (40) est pourvue d'au moins une paire d'ouvertures de came arquées (41), chaque plaquette de frein (30) étant pourvue d'une goupille d'entrée respective (32) insérée dans une ouverture de came correspondante (41).

4. Mécanisme de transmission selon la revendication 3, dans lequel le rayon de courbure de chaque ouverture de came (41) est tel que la broche d'entrée respective (32) est déplacée radialement vers l'axe de rotation de l'arbre d'entrée (4) dans la condition de rotation de l'arbre d'entrée (4), tandis que la broche d'entrée (32) est déplacée radialement dudit ressort (31) vers la surface de freinage (21) en l'absence de rotation de l'arbre d'entrée (4).

5. Mécanisme de transmission selon une ou plusieurs des revendications précédentes, dans lequel l'élément de freinage (3) est relié à l'arbre de sortie (1) au moyen d'un trou d'engagement (33) prévu dans le pivot d'articulation des patins de frein (30), l'arbre de sortie (1) étant muni d'une broche de sortie excentrique (11) insérée dans ledit trou d'engagement (33).

6. Mécanisme de transmission selon une ou plusieurs des revendications précédentes, dans lequel les patins de frein (30) sont des éléments arqués et comportent chacun une première extrémité à contrainte réciproque et une seconde extrémité libre pourvue de ladite zone de contact de freinage (34).

7. Mécanisme de transmission selon une ou plusieurs des revendications précédentes, dans lequel l'arbre d'entrée (4) et l'arbre de sortie (1) sont alignés axialement l'un par rapport à l'autre.

8. Mécanisme de transmission selon une ou plusieurs des revendications précédentes, dans lequel la surface de freinage (21) est constituée d'un élément tubulaire inséré dans la cavité cylindrique (20) et contraint à la cavité cylindrique (20) elle-même.

9. Boîte de vitesses à entrée et sortie coaxiales,
**caractérisée en ce qu'**
elle comprend au moins un mécanisme selon une ou plusieurs des revendications précédentes, lequel mécanisme est connecté en série à ladite entrée et/ou à ladite sortie.

10. Boîte de vitesses selon la revendication 9, comprenant une boîte de vitesses harmonique, laquelle boîte de vitesses harmonique comprend une cannelure flexible de sortie (51) s'engrenant à l'intérieur d'un boîtier extérieur (53) muni d'une couronne dentée intérieurement et d'un élément générateur d'ondes de déformation d'entrée (50), l'arbre de sortie (1) du mécanisme étant relié audit élément de génération d'ondes de contrainte d'entrée (50), et dans lequel le mécanisme a des dimensions telles qu'il est logé au moins en partie à l'intérieur dudit flexspline (51) et l'élément de freinage (3) est configuré pour interagir avec la surface de freinage (21), laquelle surface de freinage (21) est placée à l'intérieur dudit flexspline (51).
